(19) Europäisches Patentamt
European Patent Office
Office européen des brevets

(11) Veröffentlichungsnummer: **0 116 886**
**B1**

# (12) EUROPÄISCHE PATENTSCHRIFT

(45) Veröffentlichungstag der Patentschrift: **14.01.87**

(51) Int. Cl.⁴: **C 07 C 79/12**, C 07 C 76/02

(21) Anmeldenummer: **84101134.9**

(22) Anmeldetag: **04.02.84**

## (54) Verfahren zur Herstellung von fluorierten Nitroalkanen.

(30) Priorität: **16.02.83 DE 3305201**

(43) Veröffentlichungstag der Anmeldung: **29.08.84 Patentblatt 84/35**

(45) Bekanntmachung des Hinweises auf die Patenterteilung: **14.01.87 Patentblatt 87/3**

(84) Benannte Vertragsstaaten: **CH DE FR GB IT LI**

(56) Entgegenhaltungen:
**FR-A-892 442**
**US-A-2 864 853**
**US-A-3 118 004**

**DOKLADY AKADEMII NAUK SSR; Band 149,1963, Moskau A.I. TITOV "Der Ionenmechanismus bei der Nitrierung von ungesättigten Verbindungen. Nitrofluorierung von Olefinen und deren Halogensubstitutionsprodukten" Seiten 330-333**
**IZVESTIYA AKADEMII NAUK SSSR, SERIYA KHIMICHESKAYA 1963 I.L. KNUNYANTS, L.S. GERMAN, I.N. ROZKHOV "Aliphatische Fluoronitroverbindungen. 1. Konjugierte Nitrofluorierung von Olefinen" Seiten 1946-1950**
**CHEMICAL ABSTRACTS, Band 62, No. 12, 7. Juni 1965, Columbus, Ohio, USA I.V. MARTYNOV, YU.L. KRUGLYAK "Halo-alpha-nitro carboxylic acids. V. Reaction of chlorides of halo-alpha-nitro**

(73) Patentinhaber: **BAYER AG, Konzernverwaltung RP Patentabteilung, D-5090 Leverkusen 1 Bayerwerk (DE)**

(72) Erfinder: **Baasner, Bernd, Dr., Mozartstrasse 41, D-5090 Leverkusen 1 (DE)**
Erfinder: **Hagemann, Hermann, Dr., Kandinsky-Strasse 52, D-5090 Leverkusen 1 (DE)**
Erfinder: **Klauke, Erich, Dr., Eichendorffweg 8, D-5068 Odenthal (DE)**

(56) Entgegenhaltungen: (Fortsetzung)
**carboxylic acids with water" Zusammenfassung Nr. 14 490b**

**EP 0 116 886 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein verbessertes Verfahren zur Herstellung von α-fluorierten Nitroalkanen und -cycloalkanen durch konjugierte Nitrofluorierung der entsprechenden Olefine. Die erfindungsgemäß herstellbaren α-fluorierten Nitroalkane sind zum Teil bereits bekannt und können als Zwischenprodukte zur Herstellung von bestimmten Herbiziden verwendet werden.

Fluorierte Nitroalkane können nach verschiedenen Verfahren hergestellt werden, zum Beispiel durch Umsetzung von aliphatischen, Nitrogruppen enthaltenden Carbonsäuren mit $SP_4/BF_3$ (vgl. Tetrahedron 26, S. 5737 (1970)). Die benötigten Ausgangsstoffe müssen erst hergestellt werden, und das anschließende Fluorierungsverfahren ist außerordentlich aufwendig und für eine technische Anwendung völlig ungeeignet.

Weiterhin ist es durch Arbeiten russischer Autoren bekannt geworden, daß durch die Reaktion von Olefinen in wasserfreiem Fluorwasserstoff mit konzentrierter Salpetersäure aliphatische α-fluorierte Nitroverbindungen gemäß dem allgemeinen Reaktionsschema:

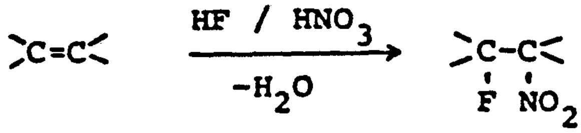


hergestellt werden können (vgl. Dokl. Akad. Nauk. SSSR 149, S. 222-5 (1963) (engl.) und Izvest. Akad. Nauk. SSSR 1963, S. 1794-7 (engl.)).

Diese Reaktion wird als konjugierte Nitrofluorierung bezeichnet und erscheint prinzipiell attraktiv für eine technische Anwendung. Es zeigt sich jedoch, daß die aus den genannten russischen Publikationen bekannten Laborvorschriften für eine direkte Übertragung in den großtechnisch-industriellen Maßstab ebenfalls völlig ungeeignet sind:

Der Fluorwasserstoff wirkt bei der vorbekannten Verfahrensweise zugleich als Fluorierungs- und als Lösungsmittel und wird in sehr großem Überschuß eingesetzt. Da bei der Reaktion in äquimolarer Menge Wasser entsteht, fällt während der Umsetzung eine wäßrige $HF/HNO_3$-Mischung an, welche auf die üblicherweise verwendeten Stahl-Rührgefäße stark korrosiv wirkt. Um diese Korrosion einzuschränken, muß ein großer HF-Überschuß verwendet werden; dadurch kann die schädliche Wasserkonzentration so stark herabgesetzt werden, daß sich sogar der Beständigkeitsbereich der betreffenden Reaktionsgefäßwerkstoffe erreichen läßt. - Bei dem bekannten Verfahren müssen auf 1 Mol Olefin 5 bis 100 Mol Fluorwasserstoff, also wenigstens 500 Mol-% Überschuß, eingesetzt werden. Die Weiterverarbeitung der enfellenden wäßrigen Säuremischung ist sehr kostenintensiv und technologisch problematisch. Eine Rückgewinnung der überschüssigen Flußsäure aus der anfallenden wäßrigen Lösung in wasserfreier Form, wie sie für erneute Umsetzungen benötigt würde, ist mit vertretbarem technischen Aufwand praktisch nicht möglich.

Es wurde nun überraschend gefunden, daß die konjugierte Nitrofluorierung auch in Gegenwart von bestimmten wasserbindenden Mitteln durchgeführt werden kann, wodurch der bei dem bekannten Verfahren notwendige große Überschuß an Fluorwasserstoff vermieden und die sonst eintretende Korrosion der als Reaktionsgefäße üblicherweise verwendeten Stahlapparaturen weitestgehend verhindert wird. Überraschend ist auch der außerordentlich glatte Reaktionsverlauf des erfindungsgemäßen Verfahrens, da die verwendeten wasserbindenden Mittel als stark reaktive Verbindungen bekannt sind und daher Neben- und Zersetzungsreaktionen hätten eingehen oder verursachen können.

Gegenstand der Erfindung ist somit ein Verfahren zur Herstellung von α-fluorierten Nitroalkanen der allgemeinen Formel

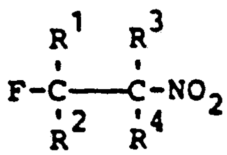
(Ia)

bzw.

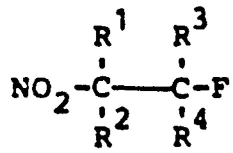
(Ib)

(I)

in welcher

$R^1$, $R^2$, $R^3$ und $R^4$ gleich oder verschieden sind und einzeln für Wasserstoff, Fluor, Chlor, Brom, Alkyl, Halogenalkyl oder Cycloalkyl stehen oder

$R^1$ und $R^3$ die angegebene Bedeutung haben und

$R^2$ und $R^4$ gemeinsam für eine Alkylengruppe mit 3 - 6 C-Atomen stehen,

durch konjugierte Nitrofluorierung der entsprechenden Olefine der Formel

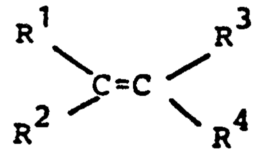

(II)

in welcher

$R^1$, $R^2$, $R^3$ und $R^4$ die oben angegebene Bedeutung haben,

mit Fluorwasserstoff und Salpetersäure, dadurch gekennzeichnet, daß die Umsetzung in Gegenwart von Fluorsulfonsäure, Schwefeltrioxid oder Thionylchlorid als wasserbindendes Mittel durchgeführt wird, wobei man auf 1 Mol Olefin der Formel (II) 1 bis 2 Mol Fluorwasserstoff einsetzt und wobei übliche Stahlapparaturen verwendet werden können.

Ob dabei die Produkte der Formel (Ia) oder (Ib) oder - in Ausnahmefällen - Gemische von (Ia) und (Ib) gebildet werden, läßt sich jeweils anhand der bekannten Markownikow-Regel ableiten.

Außerdem wurde gefunden, daß die bisher zur Verdünnung notwendige Fluorwasserstoffmenge bis auf einen molaren Überschuß von ca. 20 %, bezogen auf eingesetztes Olefin (II), vermindert werden kann, ohne daß Ausbeuteverluste eintreten.

Durch den Zusatz der oben genannten wasserentziehenden bzw. wasserbindenden Mittel wird das bei der Reaktion entstehende Wasser soweit gebunden, daß die Korrosion der üblicherweise zu verwendenden Stahlapparaturen, wie sie bei der Anwesenheit von Wasser in hohem Maße gegeben ist, erheblich vermindert oder sogar völlig ausgeschlossen wird.

Da die Umsetzung nach der Erfindung außerdem mit wesentlich geringerem HF-Überschuß (nämlich 20 Mol-% gegenüber 500 - 10.000 Mol-% bei der vorbekannten Verfahrensweise) durchgeführt werden kann, ist das neue Verfahren im Vergleich zum vorbeschriebenen erheblich kostengünstiger und technologisch problemloser durchführbar. Darüber hinaus sind bei der wasserfreien Methode zum Teil erheblich bessere Ausbeuten bei kürzerer Reaktionszeit zu erzielen. Weiterhin ist für den Fall, daß mit größeren als 20 %igen HF-Überschüssen gearbeitet wird, eine einfache Rückgewinnung des nicht verbrauchten Fluorwasserstoffs möglich. Damit kommt das erfindungsgemäße Verfahren auch für eine technische Nutzung in Betracht, wobei übliche Stahl-Rührapparaturen verwendet werden können, was einen besonderen Vorteil bedeutet.

Die als Ausgangsstoffe einzusetzenden Olefine sind durch die Formel (II) allgemein definiert. In dieser Formel stehen $R^1$, $R^2$, $R^3$ und $R^4$, die gleich oder verschieden sein können, vorzugsweise für Wasserstoff, Fluor, Chlor, Brom, Alkyl mit 1 - 4 C-Atomen, Halogenalkyl mit 1 - 4 C-Atomen (und bevorzugt mit Fluor und Chlor als Halogenatom) oder für Cycloalkyl mit 3 - 8 C-Atomen, oder es stehen $R^1$ und $R^3$ für die zuvor angegebenen Reste, und $R^2$ und $R^4$ stehen gemeinsam für eine Alkylengruppe mit 3 - 4 C-Atomen.

Die erfindungsgemäß verwendbaren Olefine sind bereits bekannt oder können nach allgemein bekannten Methoden hergestellt werden. Es können beispielsweise die im folgenden genannten Olefine als Ausgangsstoffe eingesetzt werden:

Chlorethen, 1,1-Dichlorethen, 1,1-Difluorethen, Fluorethen, Tetrafluorethen, Trifluorchlorethen, 1,2-Dichlor-1,2-difluor-ethen, Trifluorethen, 1-Chlor-1,2-difluor-ethen, 1,1-Dichlor-2-fluorethen, Ethen, 1-Chlor-1-fluorethen, 1,1-Dichlor-2,2-difluorethen, Trichlorethen, 1,2-Dichlor-1-fluorethen, Propen, 1,1-Difluorproben, 1,1-Dichlorpropen, 1-Chlor-1-fluorpropen, 2-Fluorpropen, 2-Chlorpropen, 1,1-Dichlor-3,3-dimethylpropen-1, Hexafluorpropen, 1,1,3,3,3-Pentafluorpropen, 1,1-Dichlor-2,3,3,3-tetrafluorpropen, 1,1,2-Trichlor-3,3,3-trifluorpropen, 1,1,2,3-Tetrachlor-3,3-difluorpropen, 3-Chlorpro-pen, 2,3-Dichlorpropen, 3,3,3-Trifluorpropen, Bromethen, 1,1-Difluorbuten, 1-Chlor-1-fluorbuten, 1,1-Dichlorbu-ten, Cyclchexen, 2-(Fluormethyl)-3-fluorpropen-1.

Die Reihenfolge, in der die Reaktionskomponenten zur Durchführung des Verfahrens miteinander gemischt werden, ist im prinzip beliebig. Es ist jedoch besonders zweckmäßig, in einem Stahl-Rührgefäß eine Mischung von wasserfreiem Fluorwasserstoff, konzentrierter Salpetersäure und dem betreffenden wasserentziehenden Mittel vorzulegen und das Olefin dann hinzuzufügen.

Der Überschuß an Fluorwasserstoff kann zwar im Verhältnis zum eingesetzten Olefin beliebig groß sein, jedoch genügt eine Menge von 1 - 2 Mol und - im Sinne der Erfindung bevorzugt von 1 - 1,2 Mol Fluorwasserstoff pro Mol umzusetzendes Olefin (II). Auf 1 Mol Olefin (II) werden außerdem 1 bis 2 Mol Salpetersäure eingesetzt; bevorzugt ist ein Molverhältnis von 1 bis 1,2 Mol Salpetersäure pro Mol Olefin, besonders bevorzugt von 1 Mol Salpetersäure pro Mol Olefin. Die Menge an wasserbindendem Mittel kann grundsätzlich ebenfalls beliebig groß sein, doch ist es zweckmäßig, pro Mol Olefin (II) bzw. pro Mol Salpetersäure 1 Mol bzw. 1 Äquivalent des wasserbindenden Mittels einzusetzen.

Nach dem Vorlegen des Säuregemisches wird bei Temperaturen von -80° bis +120°C, bevorzugt von -60° bis 180°C das umzusetzende Olefin in das Reaktionsgefäß gebracht, indem es eingeleitet, einkondensiert, zugetropft oder in das verschlossene Reaktionsgefäß eingedrückt wird. Die Reaktionstemperaturen selbst liegen ebenfalls zwischen -80° und +120°C, bevorzugt zwischen -20° und +80°C.

Die Reaktion erfolgt bei Normaldruck oder dem Eigendruck, der sich im Verlauf der Reaktion im verschlossenen Reaktionsgefäß aufbaut. Es kann aber auch unter höheren Drücken, etwa bis 50 bar, gearbeitet werden, wobei dieser Druck durch Aufpressen eines Inertgases (z.B. von Stickstoff) erreicht wird. Die Reaktionsdauer beträgt 0,5 bis 48 Stunden, in der Regel reicht eine Reaktionszeit von 2 bis 16 Stunden aus.

Die Aufarbeitung der Reaktionsansätze erfolgt nach bekannten Verfahrensweisen. So kann das

Reaktionsprodukt, sofern es sich als zweite Phase bereits absetzt, abgetrennt und destillativ gereinigt werden. Das Reaktionsgemisch kann aber auch mit Wasser verdännt und das Produkt mit inerten organischen Lösungsmitteln extraktiv aus dieser Rohlösung erhalten werden. Nach Neutralisation von noch vorhandenen Resten an Flußsäure und Trocknung der Extraktionslösung kann das Extraktionsmittel destillativ abgetrennt und das reine Fluornitroalkan durch anschließende Destillation bei Normaldruck oder unter vermindertem Druck gewonnen werden.

Die erfindungsgemäß herstellbaren α-Fluor-nitroalkane (I) können z.B. als Zwischenprodukte zur Herstellung von fluorhaltigen herbiziden Wirkstoffen, z.B. aus der Reihe der sym-Triazine verwendet werden. Die Nitrogruppe in den Verbindungen (I) läßt sich durch katalytische Hydrierung glatt zur Aminogruppe reduzieren, wodurch man in hohen Ausbeuten die entsprechenden α-fluorierten Alkyl- bzw. Cycloalkylamine erhält. Diese lassen sich in bekannter Weise mit Cyanurchlorid oder Cyanurfluorid zu bekannten sym-Triazinen umsetzen, welche durch entsprechende Fluoralkylaminogruppen substituiert sind und bekanntermaßen starke herbizide Eigenschaften aufweisen (vgl. z.B. DE-OS 31 27 861, DE-OS 32 18 201, DE-OS 32 18 966).

So erhält man beispielsweise ausgehend von 1-Methyl-2,2,2-trifluornitroethan auf folgendem Wege den herbiziden Wirkstoff 2-Chlor-4-ethylamino-6-(1-methyl-2,2,2-trifluorme-thyl-amino)-s-triazin:

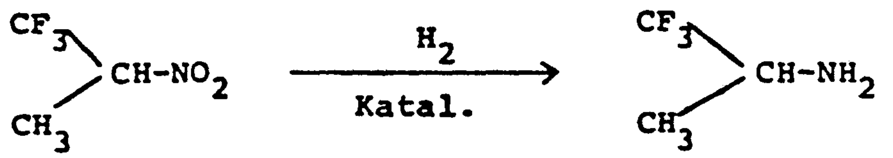


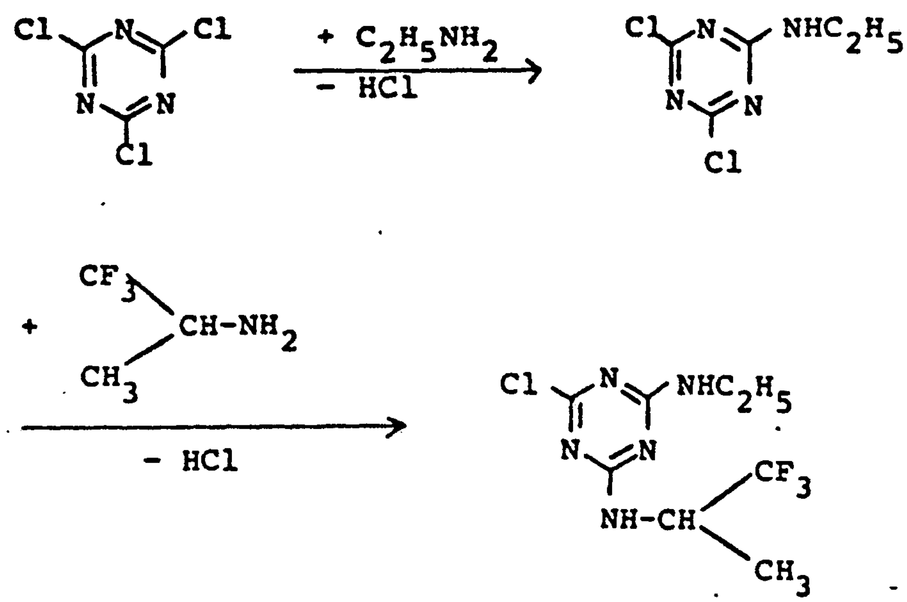


Die nachfolgenden Beispiele dienen zur weiteren Erläuterung der Erfindung.

**Beispiele**

**Beispiel 1**

$CF_3$-$CH_2$-$NO_2$

a) gemäß der Erfindung:

In einem $V_4$A-Rührgefäß wurden 220 g (11 Mol) Fluorwasserstoff, 630 g (10 Mol) Salpetersäure (d = 1,51) und 1000 g (10 Mol) Fluorsulfonsäure vorgelegt und unter Kühlung bei -10° bis +10°C 640 g (10 Mol) 1,1-Difluorethen eingeleitet. Man ließ auf Raumtemperatur kommen und 4 Stunden bei dieser Temperatur nachrühren. Anschließend wurde das Reaktionsgemisch mit 2 l Wasser verdünnt. Man trennte die organische Phase ab und extrahierte die wäßrige dreimal mit je 200 ml Dichlormethan. Die vereinigten organischen Phasen wurden mit Natriumhydrogencarbonat und Wasser neutral gewaschen, dann über Magnesiumsulfat

getrocknet. Nach Abdestillieren des Lösungsmittels erhielt man 1200 g (≙ 93 % d.Th.) 2,2,2-Trifluronitro-ethan vom Siedepunkt: 91 - 92°C; $n_D^{20}$: 1.3288. Der Korrosionswert betrug 2,3 g/m²/Tag und lag damit im optimalen Verwendungsbereich für VA-Stähle (vgl. z.B. das Handbuch "Böhler-Antinit(R)/Chemischbeständdige Stähle", Gebr. Böhler & Co. AG Edelstahlwerke, 4000 Düsseldorf-Oberkassel, Hansa-Allee 321; Tabelle G 1).

b) gemäß der Erfindung:
Analog zu Beispiel 1a) wurde ein Ansatz mit einem Zusatz von 1190 g (10 Mol) Thionylchlorid anstelle von Fluorsulfonsäure durchgeführt. Man erhielt 1120g (≙ 87 % d.Th.) 2,2,2-Trifluornitroethan; Siedepunkt: 91 - 92°C; $n_D^{20}$: 1.3292. Der Korrosionswert betrug 3,9 g/m²/Tag und lag damit ebenfalls im günstigsten Verwendungsbereich für VA-Stähle.

c) Vergleichsversuch (gemäß Izvest. Akad. Nauk. SSSR 1963, S. 1794-7 (engl.)):
In einem $V_4A$-Stahlrührautoklaven wurden 1600 g (80 Mol) Fluorwasserstoff (wasserfrei) und 630 g (10 Mol) Salpetersäure (d = 1,51) vorgelegt und unter Solekühlung bei -30° bis -10°C 640 g (10 Mol) 1,1-Difluorethen eingeleitet. Man ließ in etwa 4 Stunden auf Raumtemperatur kommen und goß das Reaktionsgemisch auf 1,5 kg Eis, trennte die organische Phase ab und extrahierte die wäßrige dreimal mit je 200 ml Dichlormethan. Die vereinigten organischen Phasen wurden mit Natriumhydrogencarbonat-Lösung und Wasser neutral gewaschen, anschließend über Magnesiumsulfat getrocknet. Nach Abdestillieren des Lösungsmittels erhielt man 790 g (≙ 61 %) 2,2,2-Tri-fluornitroethan, Siedepunkt: 91 - 92°C; $n_D^{20}$: 1.3285. Der Korrosionswert lag bei 16,4 g/m²/Tag und damit bereits im ungüstigen Anwendungsbereich für VA-Stähle.

Analog zu Beispiel 1a) wurden auch die in der folgenden Tabelle aufgeführten Verbindungen hergestellt:

Tabelle 1

| Beispiel Nr. | Olefin (II) | Produkt (I) | Siedepunkt [°C] | $n_D^{20}$ | Ausbeute (%) |
|---|---|---|---|---|---|
| 2 | $CHCl=CH_2$ | $CHClF-CH_2-NO_2$ | 58-60/45 mbar | 1.4290 | 81 |
| 3 | $CCl_2=CH_2$ | $CCl_2F-CH_2-NO_2$ | 56-7/30 mbar | 1.4380 | 92 |
| 4 | $CHF=CH_2$ | $CHF_2-CH_2-NO_2$ | 22/18 mbar | 1.3659 | 82 |
| 5 | $CCl_2=CH-CH_3$ | $CFCl_2-\underset{NO_2}{CH}-CH_3$ | 54-5/18 mbar | 1.4394 | 76 |
| 6 | $CFCl=CH-CH_3$ | $CF_2Cl-\underset{NO_2}{CH}-CH_3$ | 34-5/20 mbar | 1.3873 | 88 |
| 7 | $CF_2=CH-CH_3$ | $CF_3-\underset{NO_2}{CH}-CH_3$ | 99-100 | 1.3380 | 89 |
| 8 | $CHBr=CH_2$ | $CFBrH-CH_2-NO_2$ | 64-5/28 mbar | 1.4642 | 58 |
| 9 | $CH_3-CF=CH_2$ | $CH_3-CF_2-CH_2-NO_2$ | 40-2/25 mbar | 1.3706 | 89 |
| 10 | $CF_2=CFCl$ | $CF_3-CFCl-NO_2$ (64%) + $CF_2Cl-CF_2-NO_2$ (36%) | 36-8 | | 91 |
| 11 | $CH_2=CH_2$ | $CFH_2-CH_2-NO_2$ | 65-6/25 mbar | 1.3018 | 48 |
| 12 | $CF_3-CF=CF_2$ | $CF_3-\underset{NO_2}{CF}-CF_3$ | 16-18 | | 86 |
| 13 | $CH_3-CH=CH_2$ | $CH_3-CHF-CH_2NO_2$ | 45-6/10 mbar | | 75 |

Tabelle 1 (Fortsetzung)

| Beispiel Nr. | Olefin (II) | Produkt (I) | | Siedepunkt [°C] | $n_D^{20}$ | Ausbeute (%) |
|---|---|---|---|---|---|---|
| 14 | $CFCl=CH_2$ | $CF_2Cl-CH_2-NO_2$ | | 55-6/30 mbar | 1.3831 | 86 |
| 15 | $CF_2=CF_2$ | $CF_3-CF_2-NO_2$ | | -2 bis 0 | | 94 |
| 16 | $CF_2=CFH$ | $CF_3-CFH-NO_2$ | | 56 | 1.3010 | 86 |
| 17 | 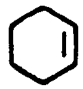 | 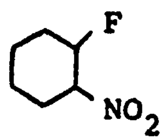 | [cis/trans] | 80-3/0,5 mbar | 1.4661 | 51 |
| 18 | 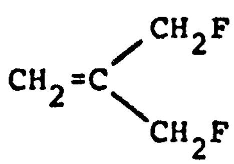 | 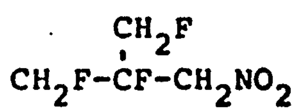 | | 83-5/20 mbar | 1.3973 | 85 |
| 19 | $CF_2=CHCl$ | $CF_3-CHCl-NO_2$ | | 79-80 | | 78 |

Die α-Fluornitroalkane der Beispiele 4, 5, 6, 8, 9, 14 und 18 sind neue Verbindungen, sind jedoch - mit Ausnahme der Verbindung 18 - Gegenstand einer Parallelanmeldung.

Die α-Fluornitroalkane der Beispiele 4, 5, 6, 8, 9, 14 und 18 sind neue Verbindungen, sind jedoch - mit Ausnahme der Verbindung 18 - Gegenstand einer Parallelanmeldung.

**Patentansprüche**

1) Verfahren zur Herstellung von α-fluorierten Nitroalkanen der allgemeinen Formel

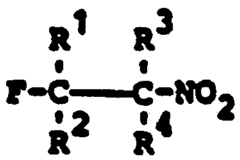
(Ia)

bzw.

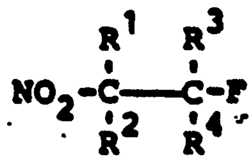
(Ib)

(I)

in welcher
$R^1$, $R^2$, $R^3$ und $R^4$ gleich oder verschieden sind und einzeln für Wasserstoff, Fluor, Chlor, Brom, Alkyl, Halogenalkyl oder Cycloalkyl stehen oder
$R^1$ und $R^3$ die angegebene Bedeutung haben und
$R^2$ und $R^4$ gemeinsam fü eine Alkylengruppe mit 3 - 6 C-Atomen stehen,
durch konjugierte Nitrofluorierung der entsprechenden Olefine der Formel

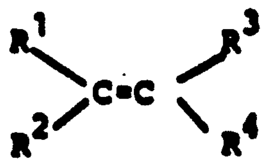

(II)

in welcher $R^1$, $R^2$, $R^3$ und $R^4$ die oben angegebene Bedeutung haben,
mit Fluorwasserstoff und Salpetersäure dadurch gekennzeichnet, daß die Umsetzung in Gegenwart von Fluorsulfonsäure, Schwefeltrioxid oder Thionylchlorid als wasserbindendes Mittel durchgeführt wird, wobei man auf 1 Mol Olefin der Formel (II) 1 bis 2 Mol Fluorwasserstoff einsetzt und wobei übliche Stahlapparaturen verwendet werden können.

2) Verfahren nach Anspruch 1, dadurch gekennzeichnet, daß man bei Temperaturen zwischen -60 und +120°C arbeitet.

3) Verfahren nach Anspruch 2, dadurch gekennzeichnet, daß man bei Temperaturen zwischen -20 und +50°C arbeitet.

4) Verfahren nach Anspruch 1, dadurch gekennzeichnet, daß man im Druckbereich von 1 bis 50 bar arbeitet.

5) Verfahren nach Anspruch 1, dadurch gekennzeichnet, daß man auf 1 Mol Olefin der Formel (II) bevorzugt 1 bis 1,2 Mol Fluorwasserstoff einsetzt.

6) Verfahren nach Anspruch 1, dadurch gekennzeichnet, daß man auf 1 Mol Olefin (II) 1 bis 2 Mol, bevorzugt 1 bis 1,2 Mol, besonders bevorzugt 1 Mol, Salpetersäure einsetzt.

7) Verfahren nach Anspruch 1, dadurch gekennzeichnet, daß man auf 1 Mol Olefin der Formel (II) bzw. 1 Mol Salpetersäure 1 Mol (äquivalent) Fluorsulfonsäure, Schwefeltrioxid oder Thionylchlorid einsetzt.

**Claims**

1. Process for the preparation of α-fluorinated nitroalkanes of the general formula

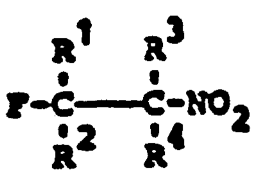
(Ia)

or

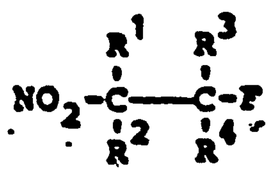
(Ib)

(I)

in which
$R^1$, $R^2$, $R^3$ and $R^4$ are identical or different and individually represent hydrogen, fluorine, chlorine, bromine, alkyl, halogenoalkyl or cycloalkyl or

$R^1$ and $R^3$ have the meaning given and
$R^2$ and $R^4$ together represent an alkylene group with 3 - 6 C atoms,
by conjugated nitrofluorination of the corresponding olefins of the formula

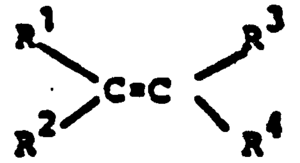

(II)

in which
$R^1$, $R^2$, $R^3$ and $R^4$ have the abovementioned meaning, with hydrogen fluoride and nitric acid, characterised in that the reaction is carried out in the presence of fluorosulphonic acid, sulphur trioxide or thionyl chloride as the water-binding agent, 1 to 2 mols of hydrogen fluoride being used to 1 mol of the olefin of the formula (II) and it being possible to use customary steel apparatuses.

2. Process according to Claim 1, characterised in that it is carried out at temperatures between -60 and +120°C.

3. Process according to Claim 2, characterised in that it is carried out at temperatures between -20 and +80°C.

4. Process according to Claim 1, characterised in that it is carried out in the pressure range of 1 to 50 bars. 5. Process according to Claim 1, characterised in that 1 to 1.2 mols of hydrogen fluoride are preferably used to 1 mol of the olefin of the formula (II).

6. Process according to Claim 1, characterised in that 1 to 2 mols, preferably 1 to 1.2 mols, particularly preferably 1 mol, of nitric acid are used to 1 mol of the olefin (II).

7. Process according to Claim 1, characterised in that 1 mol (equivalent) of fluorosulphonic acid, sulphur trioxide or thionyl chloride is used to 1 mol of the olefin of the formula (II) or 1 mol of nitric acid.

**Revendications**

1. Procédé de production de nitro-alcanes α-fluorés de formule générale

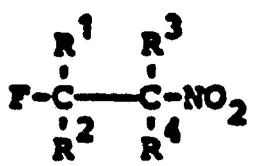

(Ia)

ou

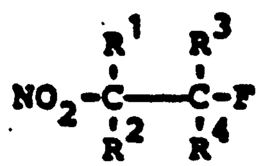

(Ib)

(I)

dans laquelle $R^1$, $R^2$, $R^3$ et $R^4$ sont identiques ou différents et représentent individuellement l'hydrogène, le fluor, le chlore, le brome, un groupe alkyle, halogénalkyle ou cycloalkyle,
ou bien
$R^1$ et $R^3$ ont la définition indiquée et
$R^2$ et $R^4$ forment conjointement un groupe alkylène de 3 à 6 atomes de carbone,
par nitrofluoration conjuguée des oléfines correspondantes de formule

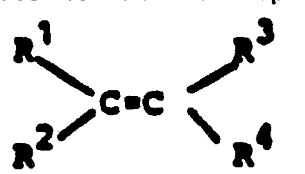

(II)

dans laquelle
$R^1$, $R^2$, $R^3$ et $R^4$ ont la définition indiquée cidessus, avec le fluorure d'hydrogène et l'acide nitrique, caractérisé en ce que la réaction est conduite en présence d'acide flourosulfonique, d'anhydride sulfurique ou de chlorure de thionyle comme agent déshydratant, et on peut alors mettre en réaction, par mole d'oléfine de formule (II) 1 à 2 moles de flourure d'hydrogène et utiliser un appareillage classique en acier. 2. Procédé suivant la revendication 1, caractérisé en ce qu'on opère à des températures comprises entre -60 et +120°C.

3. Procédé suivant la revendication 2, caractérisé en ce qu'on opére à des températures comprises entre -20 et +80°C.

4. Procédé suivant la revendication 1, caractérisé en ce qu'on opère dans un intervalle de pression de 1 à 50 bars.

5. Procédé suivant la revendication 1, caractérisé en ce qu'on utilise par mole d'oléfine de formule (II) de préférence 1 à 1,2 mole de fluorure d'hydrogène.

6. Procédé suivant la revendication 1, caractérisé en ce qu'on utilise par mole d'oléfine (II) 1 à 2 moles, de préférence 1 à 1,2 mole, notamment 1 mole, d'acide nitrique.
7. Procédé suivant la revendication 1, caractérisé en ce qu'on utilise par mole d'oléfine de formule (II) ou par mole d'acide nitrique, 1 mole (équivalent) d'acide fluorosulfonique, d'anhydride sulfurique ou de chlorure de thionyle.